(19) Europäisches Patentamt
European Patent Office
Office européen des brevets

(11) **EP 1 238 960 B1**

(12) **FASCICULE DE BREVET EUROPEEN**

(45) Date de publication et mention de la délivrance du brevet:
**17.11.2004 Bulletin 2004/47**

(51) Int Cl.⁷: **C07C 57/05**, C07C 51/16

(21) Numéro de dépôt: **02290533.5**

(22) Date de dépôt: **05.03.2002**

(54) **Procédé de fabrication d'acide acrylique à partir de propane, en l'absence d'oxygène moléculaire**

Verfahren zur Herstellung von Acrylsäure aus Propan in Abwesenheit von molekularem Sauerstoff

Process for the preparation of acrylic acid from propane in the absence of molecular oxygen

(84) Etats contractants désignés:
**BE DE FR**

(30) Priorité: **07.03.2001 FR 0103106**

(43) Date de publication de la demande:
**11.09.2002 Bulletin 2002/37**

(73) Titulaire: **Atofina**
**92800 Puteaux (FR)**

(72) Inventeur: **Dubois, Jean-Luc**
**57890 Porcelette (FR)**

(74) Mandataire: **Cabinet Hirsch**
**58, avenue Marceau**
**75008 Paris (FR)**

(56) Documents cités:
EP-A- 0 603 836          EP-A- 0 895 809
WO-A-99/03809          FR-A- 2 782 514
US-A- 4 341 717          US-A- 5 330 954
US-A- 5 780 700          US-A- 6 025 523
US-A- 6 080 893

**Description**

La présente invention concerne la production d'acide acrylique à partir de propane en l'absence d'oxygène moléculaire.

Il est connu d'après la demande de brevet européen n°EP-A-608838 de préparer un acide carboxylique insaturé à partir d'un alcane selon une réaction d'oxydation catalytique en phase vapeur en présence d'un catalyseur contenant un oxyde métallique mixte comprenant comme composants essentiels, Mo, V, Te, O, ainsi qu'au moins un élément choisi dans le groupe constitué par le niobium, le tantalum, le tungstène, le titane, l'aluminium, le zirconium, le chrome, le manganèse, le fer, le ruthénium, le cobalt, le rhodium, le nickel, le palladium, le platinum, l'antimoine, le bismuth, le bore, l'indium et le cérium, ces éléments étant présents dans des proportions bien précises. Les utilisations d'un tel catalyseur dépourvu de silicium décrites dans les exemples de ce document conduisent à de bonnes sélectivités en acide acrylique mais elles sont mises en oeuvre en présence d'air.

La demande de brevet européen n° EP-A-895809 décrit des catalyseurs à base d'oxydes comprenant du molybdène, du vanadium, du niobium, de l'oxygène, du tellure et/ou de l'antimoine, ainsi qu'au moins un autre élément tel que le fer ou l'aluminium. Ces catalyseurs peuvent être utilisés pour la conversion du propane en acide acrylique, mais seule une conversion en présence d'oxygène moléculaire est envisagée. Ils peuvent aussi comprendre un support tel que de la silice, cependant les seuls exemples de ce document relatifs à la production d'acide acrylique, à savoir les exemples 9 et 10, mettent en oeuvre des catalyseurs dépourvus de silice.

La demande de brevet européen n°EP-A-603836 se rapporte à un procédé pour préparer un catalyseur pour la production d'un nitrile. Ce catalyseur peut être un oxyde complexe comprenant du molybdène, du vanadium, du tellure, de l'oxygène ainsi qu'au moins un autre élément qui peut être - entre autres éléments - du niobium ou de l'antimoine. Dans les exemples de cette demande de brevet sont décrites les préparations de catalyseurs contenant en outre de la silice.

La demande de brevet japonais n°JP 4235153 concerne la production d'acrylonitrile à partir de propane et d'ammoniac, selon un procédé rédox.

La Demanderesse a maintenant découvert que l'on peut fabriquer de l'acide acrylique par oxydation du propane en phase gazeuse en l'absence d'oxygène moléculaire, en faisant passer un mélange gazeux de propane et de vapeur d'eau, et le cas échéant, d'un gaz inerte, sur une composition solide d'oxydes mixtes particulière, laquelle agit comme système rédox et fournit l'oxygène nécessaire à la réaction.

Les avantages de ce nouveau procédé sont les suivants :

- la limitation de la suroxydation des produits formés qui a lieu en présence d'oxygène moléculaire ; selon la présente invention, du fait que l'on opère en l'absence d'oxygène moléculaire, la formation de $CO_x$ (monoxyde de carbone et dioxyde de carbone), produits de dégradation, est réduite, ce qui permet d'augmenter la séléctivité en acide acrylique ;
- la séléctivité en acide acrylique reste bonne lorsque le taux de réduction de la composition solide augmente ;
- la composition solide, une fois qu'elle a subi une réduction et une perte progressive de son activité, est facilement régénérable par chauffage en présence d'oxygène ou d'un gaz contenant de l'oxygène après une certaine période d'utilisation ; après la régénération, le solide retrouve son activité initiale et peut être utilisé dans un nouveau cycle de réaction ;
- la séparation des étapes de réduction de la composition solide et de régénération de celle-ci permet :

- d'augmenter la sélectivité en acide acrylique; et
- d'augmenter la pression partielle en propane, une telle pression partielle d'alimentation en propane n'étant plus limitée par l'existence d'une zone explosive créée par le mélange propane + oxygène.

La présente invention a donc pour objet un procédé de fabrication de l'acide acrylique à partir de propane, qui se caractérise en ce que l'on fait passer un mélange gazeux dépourvu d'oxygène moléculaire et comprenant du propane, de la vapeur d'eau, ainsi que, le cas échéant, un gaz inerte, sur une composition solide de formule (I)

$$Mo_1V_aTe_bNb_cSi_dO_x \hspace{3cm} (I)$$

dans laquelle :

- a est compris entre 0,006 et 1, bornes incluses ;
- b est compris entre 0,006 et 1, bornes incluses ;

- c est compris entre 0,006 et 1, bornes incluses ;
- d est compris entre 0 et 3,5, bornes incluses ; et
- x est la quantité d'oxygène lié aux autres éléments et dépend de leurs états d'oxydation,

pour oxyder le propane selon la réaction rédox (1) suivante :

$$\text{SOLIDE}_{oxydé} + \text{PROPANE} \rightarrow \text{SOLIDE}_{réduit} + \text{ACIDE ACRYLIQUE} \tag{1}$$

Ce procédé permet d'obtenir une sélectivité en acide acrylique de près de 60%. En outre, si le propylène, produit secondaire, et le propane non converti sont recyclés, une sélectivité globale proche de 70% est atteinte.

D'autres caractéristiques et avantages de l'invention apparaîtront à la lecture de l'exposé qui suit et qui est illustré par des exemples.

EXPOSE DETAILLE DE L'INVENTION

Le catalyseur utilisé selon l'invention répond à la formule (I) indiquée ci-dessus.

Les oxydes des différents métaux entrant dans la composition de l'oxyde mixte de formule (I) peuvent être utilisés comme matières premières dans la préparation de cette composition, mais les matières premières ne sont pas limitées aux oxydes ; comme autres matières premières, on peut citer :

- dans le cas du molybdène, le molybdate d'ammonium, le paramolybdate d'ammonium, l'heptamolybdate d'ammonium, l'acide molybdique, les halogénures ou oxyhalogénures de molybdène tels que $MoCl_5$, les composés organométalliques du molybdène comme les alkoxydes de molybdène tels que $Mo(OC_2H_5)_5$, le molybdényle d'acétylacétone ; dans le cas du vanadium, le métavanadate d'ammonium, les halogénures ou oxyhalogénures de de vanadium tels que $VCl_4$, $VCl_5$ ou $VOCl_3$, les composés organométalliques du vanadium comme les alkoxydes de vanadium tels que $VO(OC_2H_5)_3$;
- dans le cas du tellure, l'acide tellurique ;
- dans le cas du niobium, l'acide niobique, $Nb_2(C_2O_4)_5$, le tartrate de niobium, l'hydrogéno-oxylate de niobium, le niobiate d'oxotrioxalatoammonium $\{(NH_4)_3[NbO(C_2O_4)_3] \cdot 1,5H_2O\}$, l'oxalate de niobium et d'ammonium, l'oxalate de niobium et de tartrate, les halogénures ou oxyhalogénures de nobium tels que $NbCl_3$, $NbCl_5$ et les composés organométalliques du niobium comme les alkoxydes de niobium tels que $Nb(OC_2H_5)_5$, $Nb(O-n-Bu)_5$ ;

et, d'une manière générale, tous les composés susceptibles de former un oxyde par calcination, à savoir, les sels métalliques d'acides organique, les sels métalliques d'acides minéraux, les composés métalliques complexes, etc.

La source de silicium est généralement constituée de silice colloïdale.

Conformément à des modes de réalisation particuliers, on peut préparer des compositions solides de formule (I) en mélangeant sous agitation des solutions aqueuses d'acide niobique, d'heptamolybdate d'ammonium, de métavanadate d'ammonium, d'acide tellurique, en ajoutant de préférence de la silice colloïdale, puis en précalcinant sous air à environ 300°C et en calcinant sous azote à environ 600°C.

De préférence, dans la composition solide de formule (I) :

- a est compris entre 0,09 et 0,8, bornes incluses ;
- b est compris entre 0,04 et 0,6, bornes incluses ;
- c est compris entre 0,01 et 0,4, bornes incluses ; et
- d est compris entre 0,4 et 1,6, bornes incluses.

Selon l'invention, la fabrication de l'acide acrylique est réalisée en faisant passer un mélange gazeux dépourvu d'oxygène moléculaire et comprenant du propane et de la vapeur d'eau, ainsi que, le cas échéant, un gaz inerte, sur une composition solide de formule (I) telle qu'elle a été définie ci-dessus, pour conduire la réaction rédox (1) telle qu'indiquée ci-dessus.

Généralement, la réaction rédox (1) est conduite à une température de 200 à 500°C, de préférence de 250 à 450 °C, plus préférentiellement encore, de 350 à 400°C.

La pression est généralement de $1,01.10^4$ à $1,01.10^6$ Pa (0,1 à 10 atmosphères), de préférence de $5,05.10^4$ à $5,05.10^5$ Pa (0,5-5 atmosphères).

Le temps de séjour est généralement de 0,01 à 90 secondes, de préférence, de 0,1 à 30 secondes.

Le rapport en volume propane/vapeur d'eau dans la phase gazeuse n'est pas critique et peut varier dans de larges limites.

De même, la proportion de gaz inerte, qui peut être de l'hélium, du krypton, un mélange de ces deux gaz, ou bien de l'azote, du dioxyde de carbone, etc., n'est pas non plus critique et peut aussi varier dans de larges limites.

Comme ordre de grandeur des proportions du mélange de départ, on peut citer le ratio suivant (en volumes) : propane/inerte(He-Kr)/H$_2$O (vapeur):10-20/40-50/40-50

Au cours de la réaction rédox (1), la composition solide subit une réduction et une perte progressive de son activité. C'est pourquoi, une fois que la composition solide est au moins partiellement passée à l'état réduit, on conduit la régénération de ladite composition solide selon la réaction (2) :

$$SOLIDE_{réduit} + O_2 \rightarrow SOLIDE_{oxydé} \tag{2}$$

par chauffage en présence d'oxygène ou d'un gaz contenant de l'oxygène à une température de 250 à 500°C, pendant le temps nécessaire à la réoxydation de la composition solide.

On met en général le procédé en oeuvre jusqu'à ce que le taux de réduction de la composition solide soit compris entre 10 et 40%.

Ce taux de réduction peut être surveillé au cours de la réaction par la quantité de produits obtenus. On calcule alors la quantité d'oxygène équivalente. On peut aussi le suivre par l'exothermicité de la réaction.

Après la régénération, qui peut être effectuée dans des conditions de température et de pression identiques à, ou différentes de celles de la réaction rédox, la composition solide retrouve une activité initiale et peut être utilisée dans un nouveau cycle de réaction.

On peut conduire la réaction rédox (1) et la régénération dans un réacteur classique, tel qu'un réacteur à lit fixe, un réacteur à lit fluidisé ou un réacteur à lit transporté.

On peut donc conduire la réaction rédox (1) et la régénération dans un dispositif à deux étages, à savoir un réacteur et un régénérateur qui fonctionnent simultanément et dans lesquels alternent périodiquement deux charges de composition solide ; on peut également conduire la réaction rédox (1) et la régénération dans un même réacteur en alternant les périodes de réaction et de régénération.

De préférence, la réaction rédox (1) et la régénération sont effectuées dans un réacteur à lit de catalyseur transporté.

On peut utiliser un mode de fonctionnement à un seul passage ou avec recyclage.

Selon un mode de réalisation préféré, le propylène produit comme produit secondaire et/ou le propane n'ayant pas réagi sont recyclés (ou renvoyés) à l'entrée du réacteur, c'est-à-dire qu'ils sont réintroduits à l'entrée du réacteur, en mélange ou parallèlement avec le mélange de départ de propane, de vapeur d'eau et le cas échéant de gaz inerte(s).

Exemples

Les exemples suivants illustrent la présente invention sans toutefois en limiter la portée.

Dans les formules indiquées dans les Exemples 1 et 2, x est la quantité d'oxygène lié aux autres éléments et dépend de leurs états d'oxydation.

Les conversions, sélectivités et rendements sont définis comme suit :

$$\text{Conversion (\%) du propane} = \frac{\text{Nombre de moles de propane ayant réagi}}{\text{Nombre de moles de propane introduites}} \times 100$$

$$\text{Sélectivité (\%) en acide acrylique} = \frac{\text{Nombre de moles d'acide acrylique formées}}{\text{Nombre de moles de propane ayant réagi}} \times 100$$

$$\text{Rendement (\%) en acide acrylique} = \frac{\text{Nombre de moles d'acide acrylique formées}}{\text{Nombre de moles de propane introduites}} = \times 100$$

Les sélectivités et rendements relatifs aux autres composés sont calculées de manière similaire.

Exemple 1

*Préparation du catalyseur A de formule Mo$_1$V$_{0,33}$Nb$_{0,11}$Te$_{0,22}$Si$_{1,08}$O$_x$*

a) Préparation d'une solution de niobium

Dans un bécher de 600 ml, on introduit 80 g d'eau distillée puis 6,4 g (0,038 moles) d'acide niobique. On ajoute ensuite 12,9 g (0,102 mole) d'acide oxalique dihydraté.

Le rapport molaire acide oxyalique/niobium est donc de 2,69.

On chauffe la solution obtenue précédemment à 60°C pendant 2 heures et 19 minutes, en couvrant pour éviter l'évaporation et en agitant. On obtient ainsi une suspension blanche que l'on laisse refroidir sous agitation jusqu'à 30°C , ce qui dure environ 2 heures.

b) Préparation d'une solution de Mo, V et Te

Dans un bécher de 600 ml, on introduit 265 g d'eau distillée, 61 g (0,346 mole) d'heptamolybdate d'ammonium, 13,3 g (0,114 moles) de métavanadate d'ammonium NH$_4$VO$_3$ et 17,4 g (0,076 moles) d'acide tellurique (fournisseur : FLUKA).

On chauffe la solution obtenue précédemment à 60°C pendant 1 heure et 35 minutes, en couvrant pour éviter l'évaporation et en agitant. On obtient ainsi une solution limpide rouge que l'on laisse refroidir sous agitation jusqu'à 30°C, ce qui dure environ 2 heures.

c) Introduction de la silice

Dans 56 g d'eau distillée, on introduit 56 g de silice Ludox (contenant 40% en poids de silice, fournie par la société Dupont). La solution à 20% en poids de silice obtenue est ensuite introduite sous agitation dans la solution de Mo, V et Te préparée précédemment. Cette dernière conserve sa limpidité et sa coloration rouge.

On ajoute ensuite la solution de niobium préparée précédemment. On obtient ainsi un gel orange fluo au bout de quelques minutes d'agitation. On met alors cette solution à l'étuve à 130°C pendant une nuit.

On récupère ainsi 114,9 g de précurseur qui se présente sous la forme d'un produit sec, contenant 0,373 moles de Si, ce qui correspond à un pourcentage massique d'oxyde de silicium de 22,2 %.

d) Calcination

Tout d'abord, on précalcine 30 g du précurseur obtenu précédemment pendant 4 heures à 300°C sous flux d'air de 48,2 ml/min/g de précurseur. Le solide obtenu est ensuite calciné pendant 2 heures à 600°C sous un flux d'azote de 48,1 ml/min/g de solide.

On obtient ainsi 24,9 g de catalyseur A.

Exemple 2

*Préparation du catalyseur B de formule Mo$_1$V$_{0,33}$Nb$_{0,11}$Te$_{0,22}$O$_x$*

On procède comme indiqué dans les parties a) et b) de l'Exemple 1.

Ensuite, on introduit la solution de niobium dans celle de Mo, V et Te. On obtient ainsi un gel orange fluo au bout de quelques minutes d'agitation. On met alors cette solution à l'étuve à 130°C pendant une nuit.

On récupère ainsi 88,8 g de précurseur.

On procède ensuite comme indiqué dans la partie d) de l'Exemple 1.

On obtient ainsi 20,8 g de catalyseur B.

Exemple 3

*Tests du catalyseur A*

a) Tests en mode pulsé

a1) Mode opératoire

Le mode opératoire mis en oeuvre est détaillé ci-après.

On charge dans un réacteur vertical, du bas vers le haut, une première hauteur de 2 ml (2,346 g) de carbure de silicium sous forme de particules de 1,19 mm de diamètre (tamisage entre 1 et 1,25 mm), une seconde hauteur de 5 ml (5,458 g) de catalyseur sous forme de particules de 0,25 à 1 mm, puis une troisième hauteur du même carbure de silicium que précédemment (19,547 g).

Le réacteur est ensuite chauffé à 250°C et le vaporisateur à 200°C. L'amorçage électrique de la pompe à eau est activé.

Une fois que le réacteur et le vaporisateur ont atteint les températures indiquées ci-dessus, on active la pompe à eau et on fait monter la température du réacteur à 350°C. Ensuite, on fait encore monter la température du réacteur jusqu'à 380°C par palliers de 10°C, la durée de chaque pallier étant de 10 minutes.

On laisse ensuite le réacteur se stabiliser pendant 30 minutes.

Puis, de l'oxygène est introduit en 10 impulsions de 23 secondes chacune pour bien oxyder le catalyseur. Le catalyseur est considéré comme totalement oxydé lorsque la température du point chaud s'est stabilisée, c'est-à-dire quand il n'y a plus d'exothermie due à la réaction (en suivant la température du catalyseur mesurée au moyen d'un thermocouple placé dans le lit catalytique, on peut voir les fluctuations de température en fonction des impulsions).

La pression à l'entrée du réacteur était d'environ 1,1 bar et la perte de charge à travers le réacteur est d'environ 0,1 bar.

Pour ce qui est de la production d'acide acrylique propement dite, un bilan rédox est composé de 60 cycles rédox. Un cycle rédox représente :

- 13 secondes de propane dans un flux continu d'hélium-krypton/eau,
- 45 secondes de flux continu d'hélium-krypton/eau,
- 20 secondes d'oxygène dans un flux continu d'hélium-krypton/eau,
- 45 secondes de flux continu d'hélium-krypton/eau.

Pendant le bilan, quatre prélèvements sont faits, chacun représentant 15 cycles. On effectue aussi 4 prélèvements de gaz à l'aide de poches à gaz, chaque prélèvement représentant environ 15 cycles ou seulement 13 ou 14 cycles pour la dernière poche. (Les prélèvements de gaz sont effectués sur une durée correspondant à un multiple de la durée d'un cycle, pour pouvoir connaître la quantité théorique de propane injectée).

Chaque petit flacon laveur (de 25 ml de contenance et rempli de 20 ml d'eau) est équipé d'une poche à gaz, et lorsque l'on connecte le flacon à la sortie du réacteur (dès que le liquide fait des bulles), la poche est ouverte et le chronomètre est déclenché. Un cycle dure 133 secondes et le bilan a, par conséquent, une durée de 2 heures et 13 minutes.

Pour vérifier l'état d'oxydation du catalyseur, une nouvelle série de 10 impulsions de 23 secondes d'oxygène est effectuée. Elle montre que l'état d'oxydation du solide a été maintenu pendant le bilan.

Le taux de réduction atteint correspond à une réduction de 10% de l'oxygène de surface, pour un catalyseur solide ayant une surface spécifique massique de 10m$^2$/g.

Les effluents liquides sont analysés sur un chromatographe HP 6890, après avoir effectué un étalonnage spécifique.

Les gaz sont analysés pendant le bilan sur un chromatographe micro-GC Chrompack.

Un dosage de l'acidité est effectué sur le mélange des flacons en fin de manipulation, pour déterminer le nombre exact de moles d'acide produites au cours du bilan et valider les analyses chromatographiques.

a2) Résultats

Pour étudier les performances du catalyseur dans le temps, une série de tests effectués dans les mêmes conditions a été réalisée sur plusieurs jours.

Le catalyseur a subi 10 tests en mode pulsé, ce qui permet de dire que le catalyseur est resté en contact avec du propane pendant 130 minutes, temps cumulé sur 600 impulsions.

Entre chaque bilan en mode pulsé, une série de 10 impulsions de 23 secondes d'oxygène est faite pour permettre au catalyseur de rester oxydé. En prenant en compte la durée des bilans, les séries de réoxydation et la stabilisation en température, le catalyseur est resté environ 40 heures à 380°C.

Les tests T1, T2, T3, T4 et T5 ont été réalisés comme expliqué précédemment, soit :

- le bilan de 60 cycles est découpé en quatre parties de 15 cycles ;
- 4 petits flacons laveurs de 25 ml sont utilisés pour récupérer les effluents liquides de chacune des séries de 15 cycles ; une analyse chromatographique est effectuée sur chaque flacon. Un dosage chimique d'acidité est fait sur la totalité des effluents récupérés (mélange des 4 flacons) ;
- 4 analyses de gaz sont effectuées sur toute la durée du bilan ; le gaz est prélevé pendant 15 cycles. On a donc un prélèvement gaz par petit flacon laveur.

Ainsi, on a donc 4 prélèvements liquides + gaz au cours des bilans. Les résultats présentés dans le tableau et sur le graphique correspondent à la moyenne des résultats des 4 parties (flacons et gaz).

Les bilans B1, B2, B3, B4 et B5 ont été effectués légèrement différemment :

- les effluents liquides ont été récupérés sur toute la durée du bilan dans un grand flacon de 125 ml ; une analyse chromatograhique et un dosage chimique d'acidité sont faits sur ce flacon ;
- un seul prélèvement de gaz de 15 cycles est effectué au cours du bilan.

Ainsi, une analyse liquide de tout le bilan et une analyse gaz d'une parte du bilan sont faites pour ces tests. De ce fait, les résultats sont un peu moins précis, ce qui peut expliquer que les bilans carbone soient un peu moins bons.

Pour tous les tests, les teneurs déterminées par chromatographie sont correctes puisque confirmées par le dosage chimique d'acide dans les effluents liquides récupérés.

Les résultats sont consignés dans le Tableau 1 suivant, dans lequel :

AA signifie acide acrylique ;
Aac signifie acide acétique ;
$CO_x$ signifie oxydes de carbone ;
TTU signifie taux de transformation unitaire (ou rendement).

| TEST | T1 | B1 | T2 | T3 | B2 | B3 | T4 | B4 | T5 | B5 |
|---|---|---|---|---|---|---|---|---|---|---|
| Temps cumulé sous propane(min) | 10 (13)[7] | 20 (26) | 30 (39) | 40 (52) | 50 (65) | 60 (78) | 70 (91) | 80 (104) | 90 (117) | 100 (130) |
| Point chaud (°C) | 383,7 à 384,5 | | | | | | | | | |
| Bilan carbone (%) | 98,1 | 103,1 | 100,7 | 96,5 | 98,1 | 101,1 | 98,8 | 102,9 | 98,9 | 108,0[8] |
| Conversion Propane (%)[9] | 16,8 | 17,2 | 16,8 | 16,6 | 15,7 | 17,4 | 16,6 | 17,9 | 16,8 | 18 |
| Acidité : chromatographie(moles) | $9,1 \times 10^{-4}$ | $10,3 \times 10^{-4}$ | $9,6 \times 10^{-4}$ | $9,8 \times 10^{-4}$ | $8,9 \times 10^{-4}$ | $9,6 \times 10^{-4}$ | $9,7 \times 10^{-4}$ | $10,6 \times 10^{-4}$ | $9,9 \times 10^{-4}$ | $10,5 \times 10^{-4}$ |
| Acidité : dosage chimique (moles) | $9,4 \times 10^{-4}$ | $9,7 \times 10^{-4}$ | $9,4 \times 10^{-4}$ | $9,4 \times 10^{-4}$ | $8,6 \times 10^{-4}$ | $9,9 \times 10^{-4}$ | $9,3 \times 10^{-4}$ | $10,3 \times 10^{-4}$ | $9,3 \times 10^{-4}$ | $10,1 \times 10^{-4}$ |
| **Rendement acide Acrylique (%)** | 9,6 | 9,7 | 9,5 | 9,5 | 8,6 | 10,0 | 9,3 | 10,4 | 9,3 | 10,1 |
| Rendement acide Acétique (%) | 1,1 | 1,2 | 1,1 | 1,1 | 1,0 | 1,2 | 1,1 | 1,2 | 1,2 | 1,2 |
| Rendement Propylène (%) | 3,0 | 3,0 | 3,0 | 2,8 | 2,9 | 3,0 | 2,9 | 3,0 | 2,9 | 3,1 |
| Rendement en Produits Utiles[10] (%) | 13,9 | 14,4 | 13,8 | 13,6 | 12,7 | 14,4 | 13,5 | 14,8 | 13,6 | 14,6 |
| Rendement en $CO_x$ (%) | 2,8 | 2,8 | 2,9 | 2,9 | 2,9 | 2,9 | 3,0 | 3,0 | 3,1 | 3,3 |
| **Sélectivité en acide acrylique (%)[11]** | 57,3 | 56,4 | 56,9 | 57,2 | 54,7 | 57,7 | 56,2 | 58 | 55,6 | 56 |
| Sélectivité en acide Acétique (%) | 6,4 | 7,1 | 6,4 | 6,8 | 6,5 | 6,7 | 6,9 | 6,7 | 7,1 | 6,8 |
| Sélectivité en Propylène (%) | 18 | 17,7 | 17,9 | 16,9 | 18,5 | 17,2 | 17,3 | 16,8 | 17,1 | 17,3 |
| Sélectivité en Produits utiles (%) | 82,6 | 82,5 | 82,4 | 82,2 | 80,8 | 82,8 | 81,7 | 82,8 | 81,1 | 81,3 |
| Sélectivité en $CO_x$ (%) | 16,8 | 17,1 | 17,1 | 17,4 | 18,8 | 16,8 | 18,0 | 16,7 | 18,5 | 18,3 |
| Moles d'oxygène consommé[12] | $5,8 \times 10^{-3}$ | $6,04 \times 10^{-3}$ | $5,9 \times 10^{-3}$ | $5,9 \times 10^{-3}$ | $5,6 \times 10^{-3}$ | $6,1 \times 10^{-3}$ | $5,9 \times 10^{-3}$ | $6,3 \times 10^{-3}$ | $6,0 \times 10^{-3}$ | $6,6 \times 10^{-3}$ |
| Productivité AA[13] <br> Productivité Aac <br> en moles/kg de catalyseur/h | • 0,676 <br> • 0,113 | • 0,687 <br> • 0,129 | • 0,675 <br> • 0,114 | • 0,671 <br> • 0,120 | • 0,611 <br> • 0,108 | • 0,710 <br> • 0,123 | • 0,659 <br> • 0,121 | • 0,734 <br> • 0,128 | • 0 657 <br> • 0,125 | • 0,717 <br> • 0,130 |

[7] En théorie, l'impulsion de propane est de 10s. En pratique, sa durée est de 13s. La première valeur correspond à la théorie, soit à une impulsion de 10 secondes, la deuxième valeur, indiquée entre parenthèses, à une impulsion de 13s.

[8] Le bilan carbone est excédentaire. Erreur probable sur l'analyse de gaz, la poche était poreuse.

[9] Conversion propane = somme des TTU

[10] Produits utilises : acide acrylique, acide acétique, acroléine, acétaldéhyde, acétone et propylène

[11] Les sélectivités sont déterminées en faisant : TTU produit /Σ TTU

[12] Le nombre de moles d'$O_2$ consommé est calculé sur la base des produits formés

[13] En mode rédox, la productivité est calculée en prenant en compte la durée de l'impulsion de propane (13s)

On constate qu'au fil des tests, le catalyseur est resté stable en donnant des résultats quasiment identiques à chaque test.

Les résultats sont très bons, la sélectivité en acide acrylique (AA) étant proche de 60% (elle varie de 55% à 58% sur les 10 tests) et celle en propylène (Pen) étant de 17,5%.

La conversion du propane (Pan) est de 17% en moyenne, elle varie de 16 à 18% pendant l'essai. Elle est déterminée en faisant la somme des TTU produits.

En outre, après déchargement du catalyseur, on a observé que le réacteur était propre, c'est-à-dire qu'il n'y avait pas de dépôt noir en sortie du réacteur, contrairement à ce qui produit au cours des tests co-alimentés habituels.

b) Tests en mode co-alimenté

Ces tests sont classiques, c'est-à-dire conformes aux procédés connus. Ils n'ont été effectués qu'à des fins de comparaison.

De l'air était utilisé à la place du mélange krypton-hélium pour fournir de l'oxygène moléculaire et l'alimentation était donc constituée d'un mélange de propane, d'air et de vapeur d'eau.

La pression à l'entrée du réacteur était d'environ 1,15 bar et la perte de charge à travers le réacteur était d'environ 0,15 bar.

La masse de catalyseur chargée était de 4,872 g, ce qui équivalait à une hauteur de 5 ml dans le réacteur.

Les résultats et conditions opératoires sont consignés dans le tableau 2 suivant, dans lequel :

WH signifie vitesse volumique horaire ;
AA et Aac, $CO_x$ et TTU ont les mêmes significations que dans le Tableau 1.

| TESTS | T6 | T7 |
|---|---|---|
| Mode | Co-alimenté | Co-alimenté |
| Propane/Air /H$_2$O (en volume) | 9,1/45,5/45,5 | 9,1/45,4/45,5 |
| VVH (h$^{-1}$) | 1730 | 1730 |
| Température de réaction (°C) | 390 | 400 |
| Point chaud (°C) | 408-411 | 431 |
| Durée du bilan (h) après 1h30 de stabilisation | 1h00 | 1h00 |
| Bilan carbone (%) | 105,2 | 102,4 |
| Conversion Propane (%)[9] | 22,2 | 31,2 |
| Acidité : chromatographie (moles) | n.d. | $52,1 \times 10^{-4}$ |
| Acidité : dosage chimique (moles) | n.d. | $53,8 \times 10^{-4}$ |
| **Rendement Acide Acrylique (%)** | 11,0 | 12,2 |
| Rendement acide Acétique (%) | 1,0 | 1,8 |
| Rendement Propylène (%) | 3,9 | 3,78 |
| Rendement Produits Utiles (%) | 16,0 | 17,88 |
| Rendements $CO_x$ (%) | 6,1 | 13,34 |
| **Sélectivité en acide Acrylique (%)** | 49,6 | 39,0 |
| Sélectivité en acide Acétique (%) | 4,2 | 5,8 |
| Sélectivité en Propylène (%) | 17,6 | 12,1 |
| Sélectivité en Produits utiles[14] (%) | 72,2 | 57,2 |
| Sélectivité en $CO_x$ (%) | 27,5 | 42,7 |

[9] Conversion propane = somme des TTU

[14] Produits utiles : acide acrylique, acide acétique, acroléine, acétaldéhyde, acétone et propylène

(suite)

| TESTS | T6 | T7 |
|---|---|---|
| Productivité AA[15] | • 0,796 | • 0,882 |
| Productivité Aac | • 0,072 | • 0,194 |
| en moles/kg de catalyseur/h | | |

[15] En mode rédox, la productivité est calculée en prenant en compte la durée de l'impulsion de propane (13s)

En comparant les résultats du Tableau 1 (invention) à ceux du Tableau 2 (état de la technique), on constate donc qu'à basse conversion (17 à 22%), la sélectivité en acide acrylique est de 8% plus forte en mode pulsé qu'en mode co-alimenté et la sélectivité en produits utiles est de 10% plus élevée.

A plus haute température, en mode co-alimenté, (colonne de droite du tableau 2), la conversion du propane augmente, mais la sélectivité en acide acrylique chute et celle en oxydes de carbone $CO_x$ augmente.

Exemple 4

*Tests du catalyseur B*

On a effectué des tests sur le catalyseur B comme indiqué dans l'Exemple 3, en mode co-alimenté et en mode pulsé.

La quantité de carbure de silicium chargé en premier (première hauteur) était de 2,44 g, la quantité de catalyseur B (deuxième hauteur) de 5,676 g et la quantité de carbure de silicium (troisième hauteur) de 19,617 g.

Les tailles des particules de carbure de silicium et de catalyseur étaient identiques à celles de l'Exemple 3.

En outre, afin de savoir si un recyclage du propylène produit est possible, au lieu de recycler le propylène produit, on a ajouté au propane différentes proportions de propylène au cours de la manipulation en mode pulsé.

Un débit de propylène a donc été ajouté au débit de propane constant, tous les 15 cycles, dans les proportions suivantes :

100% de propane - 0% de propylène pendant 15 cycles
100% de propane - 10% de propylène pendant 15 cycles
100% de propane - 20% de propylène pendant 15 cycles
100% de propane - 34% de propylène pendant 15 cycles

On observe que le propylène réinjecté est pratiquement totalement converti. La température de 360°C semble trop élevée pour le propylène, puisque les rendements en $CO_x$ augmentent suite à l'augmentation du débit de propylène.

On a procédé alors comme précédemment mais à une température de 340°C.

La pression à l'entrée du réacteur était d'environ 1,1 bar et la perte de charge à travers le réacteur était d'environ 0,1 bar.

Le taux de réduction du catalyseur après l'oxydation du propane était compris entre 10 et 40% de l'oxygène de surface, selon la température et la teneur en propylène.

Les rendements sont un peu moins élevés mais les sélectivités restent du même ordre de grandeur qu'à 360°C. On observe également un décroissance de la sélectivité en acide acrylique au profit de l'augmentation des sélectivités en $CO_x$ et en acide acétique. Par rapport au test à 360°C, on remarque une augmentation de la sélectivité en acétone.

On a procédé ensuite de nouveau de la même manière, mais à une température de 320°C.

La pression à l'entrée du réacteur était d'environ 1,1 bar et la perte de charge à travers le réacteur était d'environ 0,1 bar.

En ce qui concerne les rendements et sélectivités, on peut dresser le même constat que précédemment. En outre, on observe que la température de réaction n'est plus assez élevée pour convertir correctement le propane, ce qui explique les faibles rendements en produits, notamment pendant les 15 premiers cycles pendant lesquels il n'y a pas d'ajout de propylène. Pour les cycles suivants, l'augmentation des rendements en produits est majoritairement due à la conversion du propylène.

Les résultats des tests à 320°C, 340°C et 360°C sont regroupés dans le tableau 3 suivant, dans lequel :

Pen signifie propylène ;
AA et Aac, $CO_x$, TTU et VVH ont les mêmes significations que dans les Tableaux 1 et 2.

| TESTS | T8 | | | | T9 | | | | T10 | | | |
|---|---|---|---|---|---|---|---|---|---|---|---|---|
| Pan – Pen ou $O_2$/He-Kr/$H_2O$ [19] | 10-0 à 3,4[19] ou 20/45/45 | | | | 10-0 à 3,4 ou 20/45/45 | | | | 10-0 à 3,4 ou 20/45/45 | | | |
| Température de réaction (°C) | 360 | | | | 340 | | | | 320 | | | |
| Point chaud (°C) | 364,4 à 368,9 | | | | 343,9 à 347,8 | | | | 323,4 à 326,7 | | | |
| Durée du bilan (h) | 2h13 | | | | 2h13 | | | | 2h13 | | | |
| N° du flacon (15 cycles par flacon) | 1 | 2 | 3 | 4 | 1 | 2 | 3 | 4 | 1 | 2 | 3 | 4 |
| Bilan carbone (%) | 101 | 104 | 104 | 107 | 99 | 107 | 110 | 108 | 90 | 96 | 101 | 109 |
| Conversion Propane (%) | 21,1 | 11,2 | 12,6 | 13,4 | 14,9 | 0,5 | 1,4 | 6,2 | 22,0 | 7,6 | 7,2 | 2,2 |
| Conversion Propylène (%) | 0 | 97,1 | 96,9 | 97,0 | 0 | 96,7 | 95,4 | 96,0 | 0 | 97,0 | 95,0 | 94,5 |
| Sélectivité en acide Acrylique (%) | 44,4 | 46,2 | 39,8 | 34,9 | 39,7 | 43,2 | 31,0 | 34,3 | 35,1 | 33,8 | 31,5 | 30,7 |
| Sélectivité en acide Acétique (%) | 20,4 | 19,2 | 24,2 | 28,7 | 19,1 | 20,5 | 29,5 | 29,5 | 20,9 | 22,4 | 25,8 | 27,5 |
| Sélectivité en Propylène (%) | 11,5 | 9,45 | 7,2 | 5,4 | 15,4 | 11,5 | 8,0 | 6,1 | 16,1 | 12,8 | 7,9 | 6,5 |
| Sélectivité en Produits utiles (%) [17] | 78,1 | 77,0 | 73,5 | 71,4 | 77,3 | 80,1 | 74,9 | 76 | 79,8 | 77,7 | 77,6 | 79,1 |
| Sélectivité en $CO_x$ (%) | 21,3 | 22,4 | 25,8 | 28,0 | 20,6 | 18,7 | 23,5 | 22,5 | 18,5 | 20,6 | 20,1 | 18,2 |
| Productivité AA Productivité Aac en moles/kg de catalyseur/h [18] | • 0,752 • 0,682 | | | | • 0,562 • 0,630 | | | | • 0,426 • 0,513 | | | |

[17] Produits utiles : acide acrylique, acide acétique, acroléine, acétaldéhyde, acétone et propylène
[18] En mode rédox, la productivité est calculée en prenant en compte la durée de l'impulsion de propane (13s)
[19] Un débit de propylène est ajouté dans les proportions suivantes au débit de propane constant tous les 15 cycles :
100% Pan – 0% Pen pendant 15 cycles
100% Pan – 10% Pen pendant 15 cycles
100% Pan – 20% Pen pendant 15 cycles
100% Pan – 30% Pen pendant 15 cycles

Ainsi, quelle que soit la température de réaction (320°C, 340°C ou 360°C) tout le propylène ajouté est converti.

Le propylène sortant est donc considéré comme un produit et non comme un réactif.

**Revendications**

1. Procédé de fabrication de l'acide acrylique à partir de propane, **caractérisé en ce que** l'on fait passer un mélange gazeux dépourvu d'oxygène moléculaire et comprenant du propane, de la vapeur d'eau, ainsi que, le cas échéant, un gaz inerte, sur une composition solide de formule (I)

$$Mo_1V_aTe_bNb_cSi_dO_x \qquad (I)$$

dans laquelle :

- a est compris entre 0,006 et 1, bornes incluses ;
- b est compris entre 0,006 et 1, bornes incluses ;
- c est compris entre 0,006 et 1, bornes incluses ;
- d est compris entre 0 et 3,5, bornes incluses ; et
- x est la quantité d'oxygène lié aux autres éléments et dépend de leurs états d'oxydation,

pour oxyder le propane selon la réaction rédox (1) suivante :

$$SOLIDE_{oxydé} + PROPANE \rightarrow SOLIDE_{réduit} + ACIDE\ ACRYLIQUE \qquad (1)$$

2. Procédé selon la revendication 1, dans lequel, dans la composition solide de formule (I) :

- a est compris entre 0,09 et 0,8, bornes incluses ;
- b est compris entre 0,04 et 0,6, bornes incluses ;
- c est compris entre 0,01 et 0,4, bornes incluses ; et
- d est compris entre 0,4 et 1,6, bornes incluses.

3. Procédé selon la revendication 1 ou la revendication 2, caractérisé en que l'on conduit la réaction rédox (1) à une température de 200 à 500°C.

4. Procédé selon la revendication précédente, caractérisé en que l'on conduit la réaction rédox (1) à une température de 250 à 450°C.

5. Procédé selon l'une des revendications 1 à 4, **caractérisé en ce que** l'on conduit la réaction rédox (1) sous une pression de $1,01.10^4$ à $1,01.10^6$ Pa (0,1 à 10 atmosphères)

6. Procédé selon la réaction précédente, **caractérisé en ce que** l'on conduit la réaction rédox (1) sous une pression de $5,05.10^4$ à $5,05.10^5$ Pa (0,5-5 atmosphères).

7. Procédé selon l'une des revendications 1 à 6, **caractérisé en ce que** l'on conduit la réaction rédox (1) avec un temps de séjour de 0,01 à 90 secondes.

8. Procédé selon la réaction précédente, **caractérisé en ce que** l'on conduit la réaction rédox (1) avec un temps de séjour de 0,1 à 30 secondes.

9. Procédé selon l'une des revendications 1 à 8, **caractérisé en ce qu'**il est mis en oeuvre jusqu'à un taux de réduction de la composition solide compris entre 10 et 40%.

10. Procédé selon l'une des revendications 1 à 9, **caractérisé en ce qu'**une fois que la composition solide est au moins partiellement passée à l'état réduit, on conduit la régénération de ladite composition solide selon la réaction (2) :

$$\text{SOLIDE}_{\text{réduit}} + O_2 \rightarrow \text{SOLIDE}_{\text{oxydé}} \qquad (2)$$

par chauffage en présence d'oxygène ou d'un gaz contenant de l'oxygène à une température de 250 à 500°C, pendant le temps nécessaire à la réoxydation de la composition solide.

**11.** Procédé selon la revendication 10, **caractérisé en ce que** l'on conduit la réaction rédox (1) et la régénération dans un dispositif à deux étages, à savoir un réacteur et un régénérateur qui fonctionnement simultanément et dans lesquels alternent périodiquement deux charges de composition solide.

**12.** Procédé selon la revendication 10, **caractérisé en ce que** l'on conduit la réaction rédox (1) et la régénération dans un même réacteur en alternant les périodes de réaction et de régénération.

**13.** Procédé selon la revendication 10, **caractérisé en ce que** l'on conduit la réaction rédox (1) et la régénération dans un réacteur à lit transporté.

**14.** Procédé selon l'une des revendications 1 à 13, **caractérisé en ce que** le propylène produit et/ou le propane n'ayant pas réagi sont recyclés à l'entrée du réacteur.

**Claims**

**1.** Process for manufacturing acrylic acid from propane, **characterized in that** a gas mixture, which is free from molecular oxygen and comprises propane, steam as well as, optionally, an inert gas, is passed over a solid composition of formula (I)

$$\text{Mo}_1 \text{V}_a \text{Te}_b \text{Nb}_c \text{Si}_d \text{O}_x \qquad (I)$$

in which:

- a is between 0.006 and 1, including the end points;
- b is between 0.006 and 1, including the end points;
- c is between 0.006 and 1, including the end points;
- d is between 0 and 3.5, including the end points; and
- x is the quantity of oxygen bound to the other elements, and depends on their oxidation states,

in order to oxidize the propane according to the following redox reaction (1):

$$\text{SOLID}_{\text{oxidized}} + \text{PROPANE} \rightarrow \text{SOLID}_{\text{reduced}} + \text{ACRYLIC ACID} \qquad (1).$$

**2.** Process according to Claim 1, in which, in the solid composition of formula (I):

- a is between 0.09 and 0.8, including the end points;
- b is between 0.04 and 0.6, including the end points;
- c is between 0.01 and 0.4, including the end points;
- d is between 0.4 and 1.6, including the end points.

**3.** Process according to Claim 1 or Claim 2, **characterized in that** the redox reaction (1) is carried out at a temperature of 200 to 500°C.

**4.** Process according to the preceding claim, **characterized in that** the redox reaction (1) is carried out at a temperature of 250 to 450°C.

**5.** Process according to one of Claims 1 to 4, **characterized in that** the redox reaction (1) is carried out under a pressure of $1.01 \times 10^4$ to $1.01 \times 10^6$ Pa (0.1 to 10 atmospheres).

**6.** Process according to the preceding claim, **characterized in that** the redox reaction (1) is carried out under a pressure of $5.05 \times 10^4$ to $5.05 \times 10^5$ Pa (0.5 - 5 atmospheres).

**7.** Process according to one of Claims 1 to 6, **characterized in that** the redox reaction (1) is carried out with a residence time of 0.01 to 90 seconds.

**8.** Process according to the preceding claim, **characterized in that** the redox reaction (1) is carried out with a residence time of 0.1 to 30 seconds.

**9.** Process according to one of Claims 1 to 8, **characterized in that** it is implemented with a reduction factor of between 10 and 40% for the solid composition.

**10.** Process according to one of Claims 1 to 9, **characterized in that** once the solid composition has been converted at least partially into the reduced state, regeneration of the said solid composition is carried out according to the reaction (2):

$$SOLID_{reduced} + O_2 \text{ -> } SOLID_{oxidized} \qquad (2)$$

by heating in the presence of oxygen, or a gas containing oxygen, at a temperature of 250 to 500°C for the time needed to re-oxidize the solid composition.

**11.** Process according to Claim 10; **characterized in that** the redox reaction (1) and the regeneration are carried out in a device with two stages, namely a reactor and a regenerator which operate simultaneously, and in which two batches of solid composition alternate periodically.

**12.** Process according to Claim 10, **characterized in that** the redox reaction (1) and the regeneration are carried out in the same reactor, by alternating the periods of reaction and regeneration.

**13.** Process according to Claim 10, **characterized in that** the redox reaction (1) and the regeneration are carried out in a transported-bed reactor.

**14.** Process according to one of Claims 1 to 13, **characterized in that** the propylene which is produced, and/or the propane which has not reacted, are recycled to the inlet of the reactor.

**Patentansprüche**

**1.** Verfahren zur Herstellung von Acrylsäure aus Propan, **dadurch gekennzeichnet, dass** ein gasförmiges Gemisch, welches frei von molekularem Sauerstoff ist, und Propan, Wasserdampf sowie gegebenenfalls ein inertes Gas aufweist, an einer Feststoffmischung mit der Formel (I)

$$MO_1V_aTe_bNb_cSi_dO_x \qquad (I)$$

vorbeigeführt wird, wobei:

- a zwischen 0,006 und 1 liegt, Grenzen eingeschlossen;
- b zwischen 0,006 und 1 liegt, Grenzen eingeschlossen;
- c zwischen 0,006 und 1 liegt, Grenzen eingeschlossen;
- d zwischen 0 und 3,5 liegt, Grenzen eingeschlossen; und
- x die Menge an Sauerstoff ist, die an andere Elemente gebunden ist und von deren Oxidationszustand abhängt,

um das Propan gemäß der folgenden Redox-Reaktion (1) zu oxidieren:

$$Feststoff_{oxidiert} + Propan \rightarrow Feststoff_{reduziert} + Acrylsäure \qquad (1).$$

**2.** Verfahren nach Anspruch 1, wobei in der Feststoffmischung nach Formel (I) der Feststoffzusammensetzung:

- a zwischen 0,09 und 0,8 liegt, Grenzen eingeschlossen;
- b zwischen 0,04 und 0,6 liegt, Grenzen eingeschlossen;
- c zwischen 0,01 und 0,4 liegt, Grenzen eingeschlossen; und
- d zwischen 0,4 und 1,6 liegt, Grenzen eingeschlossen.

**3.** Verfahren nach Anspruch 1 oder nach Anspruch 2, **dadurch gekennzeichnet, dass** die Redox-Reaktion (1) bei einer Temperatur zwischen 200 und 500°C durchgeführt wird.

**4.** Verfahren nach dem vorangehenden Anspruch, **dadurch gekennzeichnet, dass** die Redox-Reaktion (1) bei einer Temperatur zwischen 250 und 450°C durchgeführt wird.

**5.** Verfahren nach einem der Ansprüche 1 bis 4, **dadurch gekennzeichnet, dass** die Redox-Reaktion (1) bei einem Druck zwischen $1,01 \times 10^4$ bis $1,01 \times 10^6$ Pa (0,1 bis 10 atm) durchgeführt wird.

**6.** Verfahren nach dem vorangehenden Anspruch, **dadurch gekennzeichnet, dass** die Redox-Reaktion (1) bei einem Druck zwischen $5,05 \times 10^4$ bis $5,05 \times 10^5$ Pa (0,5 bis 5 atm) durchgeführt wird.

**7.** Verfahren nach einem der Ansprüche 1 bis 6, **dadurch gekennzeichnet, dass** die Redox-Reaktion (1) mit einer Verweilzeit zwischen 0,01 und 90 Sekunden durchgeführt wird.

**8.** Verfahren gemäß der voranstehenden Reaktion, **dadurch gekennzeichnet, dass** die Redox-Reaktion (1) mit einer Verweilzeit zwischen 0,01 und 30 Sekunden durchgeführt wird.

**9.** Verfahren nach einem der Ansprüche 1 bis 8, **dadurch gekennzeichnet, dass** es bis zu einer Reduktionsrate der Feststoffmischung zwischen jeweils einschließlich 10 und 40 % realisiert wird.

**10.** Verfahren nach einem der Ansprüche 1 bis 9, **dadurch gekennzeichnet, dass**, wenn die Feststoffmischung zumindest teilweise in einen reduzierten Zustand übergegangen ist, man eine Regeneration besagter Feststoffmischung gemäß der Reaktion (2)

$$\text{Feststoff}_{\text{reduziert}} + O_2 \rightarrow \text{Feststoff}_{\text{oxidiert}} \tag{2}$$

durch eine Erhitzung auf eine Temperatur zwischen 250° und 500°C in Gegenwart von Sauerstoff oder einem Gas, das Sauerstoff beinhaltet, durchführt, über eine Zeitdauer, die zur Reoxidation der Feststoffmischung erforderlich ist.

**11.** Verfahren nach Anspruch 10, **dadurch gekennzeichnet, dass** die Redox-Reaktion (1) und die Regeneration in einer Vorrichtung mit zwei Etagen durchgeführt wird, und zwar als ein Reaktor und ein Regenerator, die gleichzeitig arbeiten können, und in denen zwei Chargen der Feststoffmischung periodisch alternieren.

**12.** Verfahren nach Anspruch 10, **dadurch gekennzeichnet, dass** die Redox-Reaktion (1) und die Regeneration im gleichen Reaktor durch Alternieren der Reaktions- und der Regenerationsphasen durchgeführt werden.

**13.** Verfahren nach Anspruch 10, **dadurch gekennzeichnet, dass** die Redox-Reaktion (1) und die Regeneration in einem Fließbettreaktor durchgeführt werden.

**14.** Verfahren nach einem der Ansprüche 1 bis 13, **dadurch gekennzeichnet, dass** das Propylen-Produkt und/oder das Propan die noch nicht reagiert sind, zum Eingang des Reaktors hin rezykliert werden.